# EUROPEAN PATENT SPECIFICATION

(11) **EP 4 119 079 B1**
(45) Date of publication and mention of the grant of the patent: **09.12.2026**
(21) Application number: 21912469.0
(22) Date of filing: 20.01.2021
(51) Int. Cl.: A61B 18/00, A61B 18/12, A61B 18/14

(54) **DATA ADJUSTMENT IN RADIO FREQUENCY OPERATION, AND RADIO FREQUENCY HOST**
DATENANPASSUNG IM FUNKFREQUENZBETRIEB UND FUNKFREQUENZHOST
RÉGLAGE DE DONNÉES DANS UNE OPÉRATION DE RADIOFRÉQUENCE, ET HÔTE DE RADIOFRÉQUENCE

(30) Priority: 31.12.2020 CN 202011638301
(43) Date of publication of application: 18.01.2023
(73) Proprietor: Hangzhou Broncus Medical Co., Ltd., Hangzhou, Zhejiang 310051 (CN)
(72) Inventor: CUI, Changjie, Hangzhou, Zhejiang 310051 (CN); XU, Hong, Hangzhou, Zhejiang 310051 (CN)
(74) Representative: HWP Intellectual Property
(86) International application number: PCT/CN2021/072956
(87) International publication number: WO 2022/141689

(56) References cited:
- WO-A1-2020/052231
- CN-A- 108 784 829
- CN-A- 111 214 288
- CN-A- 111 529 056
- US-A1- 2006 287 649
- US-A1- 2006 287 649
- US-A1- 2015 320 480
- US-A1- 2020 352 635

## Description

### TECHNICAL FIELD

Embodiments of the present invention relate to the field of electronic technology, and particularly to data adjustment in a radio frequency operation and a radio frequency host.

### BACKGROUND

In the radio frequency technology, a radio frequency operation is performed by accurately applying radio frequency energy to an object by a radio frequency host under the guidance of an image. In the radio frequency operation, the operation effect needs to be ensured, while care is taken to protect the object and operator from damage and injury.

In the prior art, during the operation of the radio frequency host, since the physical characteristics of the object keeps changing due to the action of radio frequency energy, safety problems will be caused to the objects or operation device or the radio frequency operation effect is caused to be unsatisfactory, if constant parameters are used to carry out the radio frequency operation.

### SUMMARY

None of the methods recited in the present application are claimed.

An embodiment of the present application provides a data adjustment method in a radio frequency operation and a radio frequency host. During the radio frequency operation, the radio frequency output power or a preset range for physical characteristic data of an object of the radio frequency operation is adjusted to improve the safety and effectiveness of radio frequency operation.

In an aspect, an embodiment of the present application provides a data adjustment method in a radio frequency operation, which includes steps of: acquiring set power data corresponding to a radio frequency operation, setting an output power of a radio frequency signal according to the set power data, and outputting the radio frequency signal to an object of the radio frequency operation; detecting physical characteristic data of the object in real time, and determining whether the physical characteristic data exceeds a preset range; adjusting the radio frequency output power when the physical characteristic data exceeds the preset range; and adjusting the preset range according to the physical characteristic data detected in real time in a preset period of time before a present moment when the physical characteristic data does not exceed the preset range.

The invention provides a radio frequency host, which includes an acquisition module, configured to acquire set power data corresponding to a radio frequency operation; a transmitting module, configured to set an output power of a radio frequency signal according to the set power data, and output the radio frequency signal to an object of the radio frequency operation; a detection module, configured to detect physical characteristic data of the object in real time, and determine whether the physical characteristic data exceeds a preset range; and an adjustment module, configured to adjust the radio frequency output power when the physical characteristic data exceeds the preset range, and adjust the preset range according to the physical characteristic data detected in real time in a preset period of time before a present moment when the physical characteristic data does not exceed the preset range.

The invention also provides a radio frequency host, which includes a storage and a processor, wherein the storage stores an executable program code; and the processor is coupled to the storage, and configured to call the executable program code stored in the storage, and implement the data adjustment method in a radio frequency operation as described above.

As can be known from the above embodiments of the present application, set power data corresponding to a radio frequency operation is acquired, an output power of a radio frequency signal is set according to the set power data, and the radio frequency signal is outputted physical characteristic data of an object of the radio frequency operation is detected in real time during the radio frequency operation, and whether the physical characteristic data exceeds a preset range is determined, wherein if the physical characteristic data exceeds the preset range, the radio frequency output power is adjusted, to reduce the risk of the radio frequency operation damaging the object and improve the safety of the radio frequency operation; and if the physical characteristic data does not exceed the preset range, the preset range of the physical characteristic data is adjusted, and the reasonableness of the preset range is automatically updated, to provide a more accurate data basis for subsequent radio frequency operations, and improve the reasonableness and success rate of the radio frequency operation.

The invention is defined by appended independent claims 1 and 8, embodiments are disclosed in appended dependent claims.

### BRIEF DESCRIPTION OF THE DRAWINGS

In order to describe the technical solutions according to the embodiments of the present invention or in the prior art more clearly, the drawings needed to be used in the embodiments or in the prior art will be described briefly below. Apparently, the drawings in the following description show some embodiments of the present application. Other drawings can be obtained by persons of ordinary skill in the art based on these drawings without creative efforts.
FIG. 1 is a schematic diagram showing an application scenario of a data adjustment method in a radio frequency operation provided in an embodiment of the present application;
FIG. 2 is a schematic flow chart of a data adjustment method in a radio frequency operation provided according to an embodiment of the present application;
FIG. 3 is a schematic flow chart of a data adjustment method in a radio frequency operation provided according to another embodiment of the present application;
FIG. 4 is a schematic structural diagram of a radio frequency host provided in an embodiment of the present application; and
FIG. 5 is a schematic diagram showing a hardware structure in a radio frequency host provided in an embodiment of the present application.

### DESCRIPTION OF THE EMBODIMENTS

In order to make the objects, technical solutions and advantages of the embodiments of the present invention clearer, the technical solutions according to the embodiments of the present invention will be clearly and completely described with reference to drawings in the embodiments of the present invention. Apparently, the embodiments described are merely some embodiments, but not all of the embodiments of the present application.

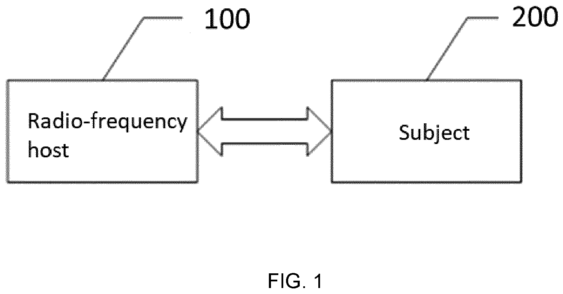

Referring to FIG. 1, a schematic diagram showing an application scenario of a data adjustment method in a radio frequency operation provided in an embodiment of the present application. The data adjustment method in a radio frequency operation includes, during the radio frequency operation, outputting a radio frequency signal at a set power, detecting physical characteristic data of an object of the radio frequency operation in real time, and determining whether to adjust the radio frequency output power or the physical characteristic data according to the change of the physical characteristic data. As a result, the data of the radio frequency operation tends to be more reasonable, to improve the success rate and safety of the radio frequency operation.

Particularly, an implementation body of the data adjustment method is a radio frequency host that may be specifically a radio frequency ablation instrument or other devices. As shown in FIG. 1, a radio frequency host 100 is connected to an object 200, and then a radio frequency operation is started, in which the radio frequency host 100 transmits a radio frequency signal to the object 200 by a radio frequency generator. In the radio frequency operation, as the nature of the object 200 changes, physical characteristic data also changes. The object 200 can be any object that needs the radio frequency operation. For example, when the radio frequency host 100 is a radio frequency ablation instrument, the object 200 can be an organism with abnormal tissues in the body that needs to be ablated.

The radio frequency host 100 has an input interface that can be externally connected to a movable storage such as U disk, or externally connected to an input device such as keyboard and mouse, to read data from the removable storage or acquire data inputted by a user from the input device. The radio frequency host 100 may also be connected to a server over a network, to obtain, from the server, big data from all radio frequency hosts connected to the server, wherein the big data includes various historical data related to the radio frequency operation.

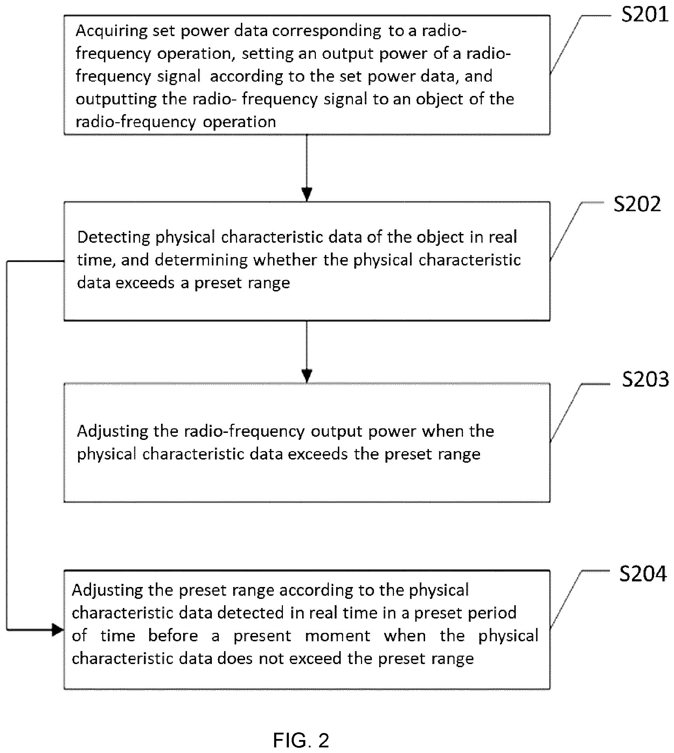

FIG. 2 is a schematic flow chart of a data adjustment method in a radio frequency operation provided in an embodiment of the present application. The method is applicable to the radio frequency host as shown in FIG. 1. As shown in FIG. 2, the method specifically includes following steps.

Step S201: acquiring set power data corresponding to a radio frequency operation, setting an output power of a radio frequency signal according to the set power data, and outputting the radio frequency signal to an object of the radio frequency operation.

Particularly, the set power data can be obtained by obtaining, from a server, historical radio frequency operation data of all radio frequency hosts in a network, or obtained from set data input into the radio frequency host by a user.

Step S202: detecting physical characteristic data of the object in real time, and determining whether the physical characteristic data exceeds a preset range,

wherein the physical characteristic data includes the temperature and impedance of the object.

In the radio frequency operation, the radio frequency signal outputted on the object has radio frequency energy, and the physical characteristic data of a site receiving the radio frequency operation will change under the action of the radio frequency energy,

The preset range is a numerical interval defined by a minimum value and a maximum value, and the way of obtaining the minimum value and the maximum value is the same as the way of obtaining the set power data in Step S201. That is, the minimum value and the maximum value can be obtained by obtaining, from a server, historical radio frequency operation data of all radio frequency hosts in a network, or obtained from set data input into the radio frequency host by a user.

Step S203: adjusting the radio frequency output power if the physical characteristic data exceeds the preset range.

If the physical characteristic data is greater than the maximum value of the preset range or less than the minimum value of the preset range, it is determined to exceed the preset range. Then, the radio frequency output power is adjusted, to reduce or increase the physical characteristic data.

In this embodiment, the calculation of the actual power detected in real time requires the measurement of the corresponding voltage and current, and then the real-time power is calculated according to a product of the voltage and current.

Step S204: adjusting the preset range according to the physical characteristic data detected in real time in a preset period of time before the present moment if the physical characteristic data does not exceed the preset range.

If the physical characteristic data does not exceed the preset range, the preset range is adjusted according to the physical characteristic data detected in real time in a preset period of time before the present moment. The adjusted physical characteristic data can be used as historical radio frequency operation data, set as a data basis for a preset range of the physical characteristic data of a next radio frequency operation, thus making the data be of great referential value, and improving the accuracy of the radio frequency operation.

In the embodiments of the present application, set power data corresponding to a radio frequency operation is acquired, an output power of a radio frequency signal is set according to the set power data, and the radio frequency signal is outputted, physical characteristic data of the object of the radio frequency operation is detected in real time during the radio frequency operation, whether the physical characteristic data exceeds a preset range is determined, wherein if the physical characteristic data exceeds the preset range, the radio frequency output power is adjusted, to reduce the risk of the radio frequency operation damaging the object and improve the safety of the radio frequency operation; and if the physical characteristic data does not exceed the preset range, the preset range of the physical characteristic data is adjusted, and the preset range is automatically adapted according to the reasonableness, to provide a more accurate data basis for subsequent radio frequency operations, and improve the reasonableness and success rate of the radio frequency operation.

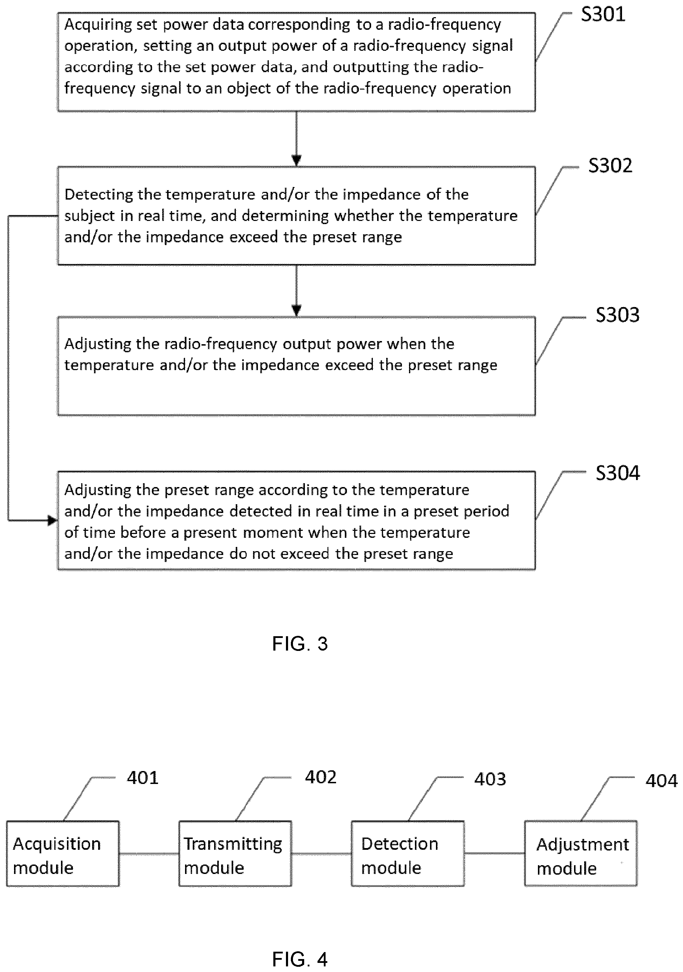

FIG. 3 is a schematic flow chart of a data adjustment method in a radio frequency operation provided in another embodiment of the present application. The method is applicable to the radio frequency host as shown in FIG. 1. As shown in FIG. 3, the method specifically includes following steps.

Step S301: acquiring set power data corresponding to a radio frequency operation, setting an output power of a radio frequency signal according to the set power data, and outputting the radio frequency signal to an object of the radio frequency operation.

Particularly, the set power data can be obtained through the following two manners.

In a first manner, historical radio frequency operation data corresponding to the task and object of the radio frequency operation is obtained from a server. Then the historical radio frequency operation data is classified according to the task of the radio frequency operation and the nature of the object. For example, the historical radio frequency operation data of task No. 1 performed on object A is classified into one category, the historical radio frequency operation data of task No. 2 performed on object is A is classified into one category, the historical radio frequency operation data of task No. 1 performed on object is B is classified into one category, and the like. Because of the same task, and the same nature of the object, the corresponding relationship between each category of historical radio frequency operation data and the radio frequency operation time is also the same.

Therefore, when the radio frequency operation is performed, radio frequency power data of corresponding historical radio frequency operation data is acquired based on the task and object of the current radio frequency operation, the acquired radio frequency power data is used as the set power data, the output power of the radio frequency signal in various periods of time of the radio frequency operation is set according to the corresponding relationship between the set power data and the radio frequency operation time, and the radio frequency signal having the output power is outputted to the object. Particularly, the output power of the radio frequency signal in the historical radio frequency operation data is determined as the set power data, wherein the set power data is specifically a change trend curve representing the corresponding relationship between the radio frequency operation time and the output power. From the change trend curve, the output power at an operation time corresponding to the current stage of the current radio frequency operation is acquired, and the acquired output power is set as the output power of the radio frequency signal.

In a second manner, the set power data can be obtained from the set data input into the radio frequency host by a user. Particularly, the set power data is acquired from the set data in the removable storage connected to the radio frequency host, or the set power data is acquired from the set data inputted via an input device of the radio frequency host. The set power data is a numerical interval including a maximum value of the set power and a minimum value of the set power.

A median value of the numerical interval is set as the output power of the radio frequency signal. The radio frequency signal having the output power is outputted to the object.

Step S302: detecting the temperature and/or the impedance of the object in real time, and determining whether the temperature and/or the impedance exceed the preset range.

Step S303: adjusting the radio frequency output power if the temperature and/or the impedance exceed the preset range.

Particularly, the radio frequency output power can be adjusted as follows. If either of the temperature or the impedance of the object detected in real time is greater than the maximum value of the preset range, the output power of the radio frequency signal is reduced to a preset first target power; and

if both the temperature and the impedance of the object detected in real time are less than the minimum value of the preset range, the output power of the radio frequency signal is increased to a preset second target power.

Due to the high temperature generated by the radio frequency energy, the impedance of the site of the object receiving the radio frequency operation is caused to increase. Accordingly, if the temperature and/or the impedance of the object detected in real time exceed the preset range, the temperature and/or the impedance generally are greater than the maximum value of the preset range, then the output power of the radio frequency signal is reduced to the preset first target power, If the temperature and/or the impedance of the object detected still exceed the preset range, the output power of the radio frequency signal is further reduced to a next target power lower than the first target power. The target power for each reduction is preset in the radio frequency host.

If a radio frequency probe provided on the radio frequency host is a multi-electrode radio frequency probe, the radio frequency output power may be adjusted as follows. If the temperature or the impedance of the object detected in real time is greater than the maximum value of the preset range, the total power needed to be set is determined according to the minimum impedance of each electrode of the multi-electrode radio frequency probe, and the real-time total power of the radio frequency probe of the radio frequency host is detected. The power adjustment amount is calculated by the default proportional integral differential (PID) algorithm according to the total power needed to be set and the real-time total power, and the target power is calculated according to the power adjustment amount and the current output power of the radio frequency signal. Then, the radio frequency output power is reduced to the target power.

Particularly, the impedances of multiple electrodes of the multi-electrode radio frequency probe are detected, and an individual electrode with the smallest impedance is determined. According to the impedance of the individual electrode, the preset power of the individual electrode, and the impedances of other electrodes of the multi-electrode radio frequency probe than the individual electrode, the powers of other electrodes are calculated, and the sum of the power of each electrode is taken as the total power needed to be set.

The power calculation formula is P = *U*² / *R*. Since each electrode of the multi-electrode radio frequency probe is connected to the same voltage output, and each electrode has the same voltage at the site of the radio frequency operation. The power of each electrode depends on the impedance R, and the power P increases with the decrease of R. The power of each individual electrode is delimited by the set total power, and can be equal to, but cannot exceed the set total power. The set total power is the sum of the power of each electrode.

Particularly, the total power needed to be set currently is calculated according to the impedance of the electrode;

According to the formula *P* = *U*² / *R*, it can be deduced that the relationship between the power Pₗᵢₘ of the electrode with the smallest impedance and the power Pₙ of other individual electrodes is:
$\frac{{P}_{lim}}{{R}_{n}} = \frac{{U}^{2} {/ R}_{lim}}{{U}^{2} {/ R}_{n}} = \frac{{R}_{n}}{{R}_{lim}} ,$ that is, ${P}_{n} = \frac{{P}_{lim} {R}_{lim}}{{R}_{n}}$.

Pₗᵢₘ is the power of the known electrode with the smallest impedance, and according to *R*ₗᵢₘ and the impedances *R*ₙ of other individual electrodes, Pₙ corresponding to each individual electrode can be obtained. The total power P needed to be set is calculated by the formula $P = {\sum }_{i = 1}^{n} {P}_{i}$.

According to the currently measured real-time total power and the total power P needed to be set, the total power increment Δ*P* can be obtained according to the PID algorithm. The PID algorithm is accomplished by
Formula 1: $u \left(k\right) = {K}_{P} \left\{err\left(k\right)+\frac{T}{{T}_{I}}{\sum }_{j = 0}^{k} err \left(j\right) + \frac{{T}_{D}}{T} \left[err\left(k\right)-err\left(k-1\right)\right]\right\} ;$ or Formula 2: $u \left(k\right) = {K}_{P} err \left(k\right) + {K}_{I} {\sum }_{j = 0}^{k} err \left(j\right) + {K}_{D} \left[err\left(k\right)-err\left(k-1\right)\right] ;$
wherein *K_{P}*, ${K}_{I} = {K}_{P} \frac{T}{{T}_{I}}$, and ${K}_{D} = {K}_{P} \frac{{T}_{D}}{T}$ are respectively the proportional coefficient, integral coefficient and differential coefficient of the PID algorithm, *T* is the sampling time, *T*_{I} is the integration time (also referred to as the integral coefficient), *T*_{D} is the differential time (also referred to as the differential coefficient), *err*(*k*) is the difference between the total power needed to be set and the real-time total power, and *u*(*k*) is the output.

By using the incremental PID algorithm ΔP = *u*(*k*)-*u*(*k* -1), it can be obtained from Formula 2 above:
$Δ P = {K}_{P} \left[err\left(k\right)-err\left(k-1\right)\right] + {K}_{I} err \left(k\right) + {K}_{D} \left[err\left(k\right)-2err\left(k-1\right)+err\left(k-2\right)\right]$

The output adjustment is calculated according to Δ*P*, and the adjustment has a one-to-one mapping relationship with Δ*P*, because the power adjustment is achieved by controlling a voltage signal from a power board, the output voltage corresponds to an input digital signal of a digital-to-analog converter, and the adjustment amount actually corresponds to this digital signal. The mapping relationship enables a corresponding relationship between the output and Δ*P*, for example, the output of 1 means that the corresponding power increment Δ*P* is 0.1 w. In this way, the control of Δ*P* is achieved according to the mapping relationship.

The current power is increased by a value of Δ*P* to obtain the target power. When Δ*P* is a negative value, the increment Δ*P* means to reduce the radio frequency output power, to lower the temperature. Otherwise, when Δ*P* is a positive value, it means to increase the radio frequency output power, to increase the temperature.

The radio frequency output power is adjusted to the target power and then outputted.

If the temperature or the impedance of the object detected in real time is less than the minimum value of the preset range, the power is adjusted in the way of that has described above.

Step S304: adjusting the preset range according to the temperature and/or the impedance detected in real time in a preset period of time before a present moment if the temperature and/or the impedance does not exceed the preset range.

Particularly, according to the various temperatures and/or impedances detected in real time in the preset period of time, and a default selection algorithm, target values are selected from various temperatures and/or the impedances in the preset time to update the extreme values of the preset range, wherein the extreme values include a minimum and a maximum value.

More specifically, the preset period of time is 10 sec. Taking temperature value as an example, a minimum value among various temperature values in 10 seconds before the present moment is selected as the minimum value of the preset range, and a maximum value among various temperature values is selected as the maximum value of the preset range; Alternatively, a median value of various temperature values in 10 seconds before the present moment is calculated, and the to-be-updated extreme values corresponding to the median value is calculated according to the median value with reference to the difference between the median value and the extreme values of the preset range before updating, wherein calculated extreme values are the extreme values of the updated preset range.

In the embodiments of the present application, set power data corresponding to a radio frequency operation is acquired, an output power of a radio frequency signal is set according to the set power data, and the radio frequency signal is outputted, the temperature and impedance of an object of the radio frequency operation are detected in real time during the radio frequency operation, and whether the temperature and/or the impedance exceed a preset range is determined, wherein if the temperature or impedance is greater than the maximum value of the preset range, the output power of the radio frequency signal is reduced, to reduce the risk of the radio frequency operation damaging the object and improve the safety of the radio frequency operation; if the temperature and impedance are both lower than the minimum value of the preset range, the output power of the radio frequency signal is increased, to improve the effect of the radio frequency operation; and further, if the temperature and/or the impedance do not exceed the preset range, the preset range is automatically updated with reasonableness, to provide a more accurate data basis for subsequent radio frequency operations, and improve the reasonableness and success rate of the radio frequency operation.

FIG. 4 is a schematic structural diagram of a radio frequency host provided in an embodiment of the present application. For ease of description, only the parts relevant to the embodiments of the present application are shown. The radio frequency host is a radio frequency host for implementing the data adjustment method in a radio frequency operation described in the above embodiments. The radio frequency host includes:
an acquisition module 401, configured to acquire set power data corresponding to a radio frequency operation;
a transmitting module 402, configured to set an output power of a radio frequency signal according to the set power data, and output the radio frequency signal to an object of the radio frequency operation;
a detection module 403, configured to detect physical characteristic data of the object in real time, and determine whether the physical characteristic data exceeds a preset range; and
an adjustment module 404, configured to adjust the radio frequency output power if the physical characteristic data exceeds the preset range, and
the adjustment module 404 further configured to adjust the preset range according to the physical characteristic data detected in real time in a preset period of time before a present moment if the physical characteristic data does not exceed the preset range.

The various modules in the radio frequency host serve to implement the following functions. Set power data corresponding to a radio frequency operation is acquired, an output power of a radio frequency signal is set according to the set power data, and the radio frequency signal is outputted, physical characteristic data of the object of the radio frequency operation is detected in real time during the radio frequency operation, and whether the physical characteristic data exceeds a preset range is determined, wherein if the physical characteristic data exceeds the preset range, the radio frequency output power is adjusted, to reduce the risk of the radio frequency operation damaging the object and improve the safety of the radio frequency operation; if the physical characteristic data does not exceed the preset range, the preset range of the physical characteristic data is adjusted, and the preset range is automatically updated according to the reasonableness, to provide a more accurate data basis for subsequent radio frequency operations, and improve the reasonableness and success rate of the radio frequency operation.

Further, the detection module 403 is further configured to detect the temperature and/or the impedance of the object in real time.

Further, the adjustment module 404 is further configured to reduce the radio frequency output power to a preset first target power if the temperature or impedance of the object detected in real time is greater than the maximum value of the preset range; and increase the radio frequency output power to a preset second target power if the temperature and the impedance of the object detected in real time are both less than the minimum value of the preset range.

If a radio frequency probe provided on the radio frequency host is a multi-electrode radio frequency probe, the detection module 403 is further configured to determine the total power needed to be set according to the minimum impedance of the electrode of the multi-electrode radio frequency probe if the temperature or the impedance of the object detected in real time is greater than the maximum value of the preset range, and
detect the real-time total power of the radio frequency probe of the radio frequency host; and
the adjustment module 404 is further configured to calculate a power adjustment amount by default PID algorithm according to the total power needed to be set and the real-time total power, calculate a target power according to the power adjustment amount and the present output power of the radio frequency signal, and reduce the radio frequency output power to the target power.

The adjustment module 403 is also configured to select target values from various temperature values and/or impedance values to update extreme values of the preset range according to the various temperatures and/or the impedances detected in real time in the preset period of time and a default selection algorithm.

The acquisition module 401 is further configured to acquire historical radio frequency operation data corresponding to the task and object of the radio frequency operation; and determine the output power of the radio frequency signal in the historical radio frequency operation data as the set power data, wherein the set power data is a change trend curve representing the corresponding relationship between the radio frequency operation time and the output power.

The transmitting module 402 is further configured to acquire the output power in an operation time corresponding to the current stage of the current radio frequency operation, and set the acquired output power as the output power of the radio frequency signal.

The acquisition module 401 is further configured to acquire the set power data from an externally connected removable storage, or acquire the set power data inputted from an input device, wherein the set power data is a numerical interval including a maximum value of the set power and a minimum value of the set power.

The transmitting module 402 is further configured to set a median value of the numerical interval as the output power of the radio frequency signal.

In the embodiments of the present application, set power data corresponding to a radio frequency operation is acquired, an output power of a radio frequency signal is set according to the set power data, and the radio frequency signal is outputted, the temperature and the impedance of an object of the radio frequency operation is detected in real time during the radio frequency operation, and whether the temperature and/or the impedance exceed a preset range is determined, wherein if the temperature or the impedance is greater than the maximum value of the preset range, the radio frequency output power is reduced, to reduce the risk of the radio frequency operation damaging the object and improve the safety of the radio frequency operation; if the temperature and impedance are both lower than the minimum value of the preset range, the output power of the radio frequency signal is increased, to improve the effect of the radio frequency operation; and further, if the temperature and/or the impedance does not exceed the preset range, the preset range is automatically updated reasonably, to provide a more accurate data basis for subsequent radio frequency operations, and improve the reasonableness and success rate of the radio frequency operation.

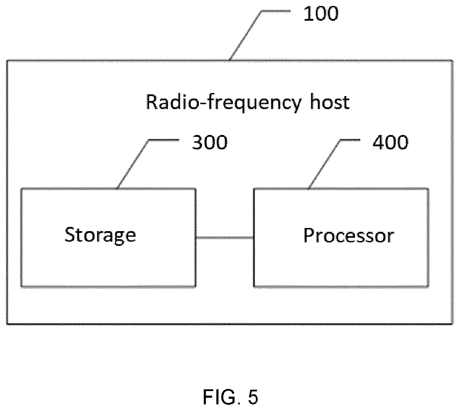

Further, as shown in FIG. 5, an embodiment of the present application also provides a radio frequency host, which includes a storage 300 and a processor 400, wherein the processor 400 may be a central processor in the radio frequency host provided in the above embodiments. The storage 300 is, for example, hard drive storage, a non-volatile storage (such as flash memory or other storages that are used to form solid-state drives and are electronically programmable to confine the deletion, etc.), and a volatile storage (such as static or dynamic random access storage), which is not limited in the embodiments of the present application.

The storage 300 stores an executable program code; and the processor 400 is coupled to the storage 300, and configured to call the executable program code stored in the storage, and implement the data adjustment method in a radio frequency operation as described above.

Further, an embodiment of the present application further provides a computer-readable storage medium. which can be provided in the radio frequency host in each of the above embodiments, and may be the storage 300 in the embodiment shown in FIG. 5. A computer program is stored in the computer-readable storage medium, and when the program is executed by a processor, the data adjustment method in a radio frequency operation according to the embodiments shown in FIG. 2 and FIG. 3 is implemented. Further, the computer-readable storage medium may also be a U disk, a removable hard disk, a read-only storage (ROM, Read-Only Memory), RAM, a magnetic disk or an optical disk and other media that can store program codes.

In the above embodiments, emphasis has been placed on the description of various embodiments. Parts of an embodiment that are not described in detail may be found in the description of other embodiments.

The data adjustment method in a radio frequency operation and the radio frequency host provided in the present invention have been described above. Changes can be made to the specific implementation and the scope of the present application by those skilled in the art according to the idea of the embodiments of the present invention. Therefore, the disclosure of this specification should not be construed as a limitation of the present invention.

## Claims

1. A radio frequency host, comprising:
an acquisition module (401), configured to acquire set power data corresponding to a radio frequency operation;
a transmitting module (402), configured to set an output power of a radio frequency signal according to the set power data, and output the radio frequency signal to an object of the radio frequency operation;
a detection module (403), configured to detect physical characteristic data of the object in real time, and determine whether the physical characteristic data exceeds a preset range, wherein the physical characteristic data comprises temperature and/or the impedance of the object, and the preset range is a numerical interval defined by a minimum value and a maximum value; and
an adjustment module (404), configured to adjust the radio frequency output power when the physical characteristic data exceeds the preset range, wherein when the physical characteristic data is greater than the maximum value of the preset range or less than the minimum value of the preset range, it is determined to exceed the preset range, and
select target values from various temperature values and/or impedance values to update the maximum value and minimum value of the preset range according to the physical characteristic data detected in real time in a preset period of time before a present moment and a default selection algorithm when the physical characteristic data does not exceed the preset range.

2. The radio frequency host according to claim 1, wherein the adjustment module (404) is further configured to reduce the radio frequency output power to a preset first target power when the temperature or impedance of the object detected in real time is greater than the maximum value of the preset range; and increase the radio frequency output power to a preset second target power when the temperature and the impedance of the object detected in real time are both less than the minimum value of the preset range.

3. The radio frequency host according to claim 1, wherein when a radio frequency probe provided on the radio frequency host is a multi-electrode radio frequency probe, the detection module (403) is further configured to determine the total power needed to be set according to the minimum impedance of the electrode of the multi-electrode radio frequency probe if the temperature or the impedance of the object detected in real time is greater than the maximum value of the preset range, and
detect the real-time total power of the radio frequency probe of the radio frequency host; and the adjustment module (404) is further configured to calculate a power adjustment amount by default PID algorithm according to the total power needed to be set and the real-time total power, calculate a target power according to the power adjustment amount and the present output power of the radio frequency signal, and reduce the radio frequency output power to the target power.

4. The radio frequency host according to claim 1, wherein the acquisition module (401) is further configured to acquire historical radio frequency operation data corresponding to the task and object of the radio frequency operation; and determine the output power of the radio frequency signal in the historical radio frequency operation data as the set power data, wherein the set power data is a change trend curve representing the corresponding relationship between the radio frequency operation time and the output power.

5. The radio frequency host according to claim 4, wherein the transmitting module (402) is further configured to acquire the output power in an operation time corresponding to the current stage of the current radio frequency operation, and set the acquired output power as the output power of the radio frequency signal.

6. The radio frequency host according to claim 1, wherein the acquisition module (401) is further configured to acquire the set power data from an externally connected removable storage, or acquire the set power data inputted from an input device, wherein the set power data is a numerical interval including a maximum value of the set power and a minimum value of the set power.

7. The radio frequency host according to claim 1, wherein the transmitting module (402) is further configured to set a median value of the numerical interval as the output power of the radio frequency signal.

8. A radio frequency host, comprising:
a storage (300) and a processor (400), wherein
the storage (300) stores an executable program code; and
the processor (400) is coupled to the storage (300), and configured to call the executable program code stored in the storage (300), and implement a data adjustment method comprising the steps of:
acquiring set power data corresponding to the radio frequency operation, setting an output power of a radio frequency signal according to the set power data, and outputting the radio frequency signal to an object of the radio frequency operation;
detecting physical characteristic data of the object in real time, and determining whether the physical characteristic data exceeds a preset range, wherein the physical characteristic data comprises temperature and/or an impedance of the object, and the preset range is a numerical interval defined by a minimum value and a maximum value;
adjusting the radio frequency power when the physical characteristic data exceeds the preset range, wherein when the physical characteristic data is greater than the maximum value of the preset range or less than the minimum value of the preset range, it is determined to exceed the preset range; and
selecting target values from various temperature values and/or impedance values to update extreme values of the preset range according to the physical characteristic data detected in real time in a preset period of time before a present moment and a default selection algorithm when the physical characteristic data does not exceed the preset range.

9. The radio frequency host according to claim 8, wherein the step of adjusting the radio frequency power when the physical characteristic data exceeds the preset range comprises steps of:
reducing the radio frequency output power to a preset first target power when the temperature or the impedance of the object detected in real time is greater than the maximum value of the preset range; and
increasing the radio frequency output power to a preset second target power when the temperature and the impedance of the object detected in real time are both less than the minimum value of the preset range.

10. The radio frequency host according to claim 8, wherein when a radio frequency probe of the radio frequency host is a multi-electrode radio frequency probe, the step of adjusting the radio frequency output power when the physical characteristic data exceeds the preset range comprises steps of:
determining the total power needed to be set according to the minimum impedance of the electrode of the multi-electrode radio frequency probe when the temperature or the impedance of the object detected in real time is greater than the maximum value of the preset range;
detecting a real-time total power of the radio frequency probe of the radio frequency host, calculating a power adjustment amount by default PID algorithm according to the total power needed to be set and the real-time total power, and calculating the target power according to the power adjustment and the present output power of the radio frequency signal; and
reducing the radio frequency output power to the target power.

11. The radio frequency host according to claim 8, wherein the step of the adjusting the preset range according to the physical characteristic data detected in real time in a preset period of time before a present moment comprises:
selecting target values from various temperature values and/or impedance values to update extreme values of the preset range according to the various temperature values and/or impedance values detected in real time in the preset period of time and a default selection algorithm.

12. The radio frequency host according to any one of claims 8 to 11, wherein the step of acquiring set power data corresponding to the radio frequency operation comprises steps of:
acquiring historical radio frequency operation data corresponding to the task and object of the radio frequency operation; and
determining the output power of the radio frequency signal in the historical radio frequency operation data as the set power data, wherein the set power data is a change trend curve representing ta corresponding relationship between the radio frequency operation time and the output power.

13. The radio frequency host according to claim 12, wherein the step of setting an output power of a radio frequency signal according to the set power data comprises:
acquiring the output power in an operation time corresponding to the current stage of the current radio frequency operation from the change trend curve, and setting the acquired output power as the output power of the radio frequency signal.

14. The radio frequency host according to any one of claims 8 to 11, wherein the step of acquiring set power data corresponding to the radio frequency operation comprises:
acquiring the set power data from an externally connected removable storage, or acquiring the set power data inputted from an input device, wherein the set power data is a numerical interval including a maximum value of the set power and a minimum value of the set power.

## Patentansprüche

1. Ein Funkfrequenzhost, umfassend:
ein Erfassungsmodul (401), das konfiguriert ist, um eingestellte Leistungsdaten entsprechend einem Funkfrequenzbetrieb zu erfassen;
ein Übertragungsmodul (402), das konfiguriert ist, um eine Ausgangsleistung eines Funkfrequenzsignals gemäß der eingestellten Leistungsdaten anzupassen, und das Funkfrequenzsignal an ein Objekt des Funkfrequenzbetriebs auszugeben,
ein Erkennungsmodul (403), das konfiguriert ist, um physikalische Eigenschaftsdaten des Objekts in Echtzeit zu erkennen, und zu bestimmen, ob die physikalischen Eigenschaftsdaten einen voreingestellten Bereich überschreiten, wobei die physikalischen Eigenschaftsdaten Temperatur und/oder die Impedanz des Objekts umfassen, und der voreingestellte Bereich ein numerisches Intervall ist, das durch einen Minimalwert und einen Maximalwert definiert wird; und
ein Anpassungsmodul (404), das konfiguriert ist, um die Funkfrequenzausgangsleistung anzupassen, wenn die physikalischen Eigenschaftsdaten den voreingestellten Bereich überschreiten, wobei dann, wenn die physikalischen Eigenschaftsdaten höher als der Maximalwert des voreingestellten Bereichs oder niedriger als der Minimalwert des voreingestellten Bereichs sind, bestimmt wird, dass der voreingestellte Bereich überschritten wird und Zielwerte aus verschiedenen Temperaturwerten und/oder Impedanzwerten ausgewählt werden, um den Maximalwert und Minimalwert des voreingestellten Bereichs gemäß den physikalischen Eigenschaftsdaten, die in Echtzeit in einem voreingestellten Zeitraum vor einem gegenwärtigen Moment erkannt wurden, und einem Standardauswahlalgorithmus zu aktualisieren, wenn die physikalischen Eigenschaftsdaten den voreingestellten Bereich nicht überschreiten.

2. Der Funkfrequenzhost gemäß Anspruch 1, wobei das Anpassungsmodul (404) des Weiteren so konfiguriert ist, dass es die Funkfrequenz-Ausgangsleistung auf eine voreingestellte erste Zielleistung reduziert, wenn die Temperatur oder Impedanz des in Echtzeit erfassten Objekts höher ist als der Maximalwert des voreingestellten Bereichs; und die Funkfrequenz-Ausgangsleistung auf eine voreingestellte zweite Zielleistung erhöht, wenn die Temperatur und Impedanz des in Echtzeit erfassten Objekts beide niedriger sind als der Minimalwert des voreingestellten Bereichs.

3. Der Funkfrequenzhost gemäß Anspruch 1, wobei dann, wenn eine an dem Funkfrequenzhost bereitgestellte Funkfrequenzsonde eine Mehrfach-Elektroden-Funkfrequenzsonde ist, das Erkennungsmodul (403) des Weiteren so konfiguriert ist, dass es die Gesamtleistung bestimmt, die gemäß der Mindestimpedanz der Elektrode der Mehrfach-Elektroden-Funkfrequenzsonde einzustellen ist, wenn die Temperatur oder Impedanz des in Echtzeit erfassten Objekts höher ist als der Maximalwert des voreingestellten Bereichs, und
die Echtzeit Gesamtleistung der Funkfrequenzsonde des Funkfrequenzhosts erkennt; und das Anpassungsmodul (404) ist des Weiteren so konfiguriert, dass es eine Höhe einer Leistungsanpassung durch einen Standard-PID-Algorithmus gemäß der einzustellenden Gesamtleistung und der Gesamtleistung in Echtzeit berechnet, eine Zielleistung gemäß der Höhe der Leistungsanpassung und der gegenwärtigen Ausgangsleistung des Funkfrequenzsignals berechnet, und die Funkfrequenz-Ausgangsleistung auf die Zielleistung reduziert.

4. Der Funkfrequenzhost gemäß Anspruch 1, wobei das Erfassungsmodul (401) des Weiteren so konfiguriert ist, dass es historische Funkfrequenz-Betriebsdaten entsprechend der Aufgabe und dem Objekt des Funkfrequenzbetriebs erfasst; und so, dass es die Ausgangsleistung des Funkfrequenzsignals in den historischen Funkfrequenz-Betriebsdaten als die eingestellten Leistungsdaten bestimmt, wobei es sich bei den eingestellten Leistungsdaten um eine Trendänderungskurve handelt, die das entsprechende Verhältnis zwischen der Funkfrequenzbetriebszeit und der Ausgangsleistung darstellt.

5. Der Funkfrequenzhost gemäß Anspruch 4, wobei das Übertragungsmodul (402) des Weiteren so konfiguriert ist, dass es die Ausgangsleistung bei einer Betriebszeit erfasst, die dem aktuellen Stand des aktuellen Funkfrequenzbetriebs entspricht, und die erfasste Ausgangsleistung als die Ausgangsleistung des Funkfrequenzsignals einstellt.

6. Der Funkfrequenzhost gemäß Anspruch 1, wobei das Erfassungsmodul (401) des Weiteren so konfiguriert ist, dass es die eingestellten Leistungsdaten von einem extern verbundenen, entfernbaren Speicher erfasst, oder die eingestellten Leistungsdaten, die von einem Eingabegerät eingegeben sind, erfasst, wobei es sich bei den eingestellten Leistungsdaten um ein numerisches Intervall handelt, einschließlich einem Maximalwert der eingestellten Leistung und einem Minimalwert der eingestellten Leistung.

7. Der Funkfrequenzhost gemäß Anspruch 1, wobei das Übertragungsmodul (402) so konfiguriert ist, dass es einen Mittelwert des numerischen Intervalls als die Ausgangsleistung des Funkfrequenzsignals einstellt.

8. Ein Funkfrequenzhost, umfassend:
einen Speicher (300) und einen Prozessor (400), wobei
der Speicher (300) einen ausführbaren Programmcode speichert; und
der Prozessor (400) mit dem Speicher (300) gekoppelt ist, und konfiguriert ist, um den in dem Speicher (300) gespeicherten ausführbaren Programmcode abzurufen, und ein Datenanpassungsverfahren durchzuführen, das die folgenden Schritte umfasst:
Erfassen von dem Funkfrequenzbetrieb entsprechenden eingestellten Leistungsdaten, Einstellen einer Ausgangsleistung des Funkfrequenzsignals gemäß der eingestellten Leistungsdaten, und Ausgeben des Funkfrequenzsignals an ein Objekt des Funkfrequenzbetriebs;
Erkennen von physikalischen Eigenschaftsdaten des in Echtzeit erfassten Objekts, und Bestimmen, ob die physikalischen Eigenschaftsdaten einen voreingestellten Bereich überschreiten, wobei die physikalischen Eigenschaftsdaten Temperatur und/oder eine Impedanz des Objekts umfassen, und bei dem voreingestellten Bereich handelt es sich um ein numerisches Intervall, das durch einen Mindestwert und einen Maximalwert definiert ist;
Anpassen der Funkfrequenzleistung, wenn die physikalischen Eigenschaftsdaten den voreingestellten Bereich überschreiten, wobei wenn die physikalischen Eigenschaftsdaten größer sind als der Maximalwert des voreingestellten Bereichs oder kleiner sind als der Minimalwert des voreingestellten Bereichs, festgelegt wird, dass der voreingestellte Bereich überschritten wird; und
Auswählen von Zielwerten aus verschiedenen Temperaturwerten und/oder Impedanzwerten, um Extremwerte des voreingestellten Bereichs gemäß den physikalischen Eigenschaftsdaten, die in Echtzeit in einem voreingestellten Zeitraum vor einem gegenwärtigen Moment erkannt wurden, und einem Standardauswahlalgorithmus zu aktualisieren, wenn die physikalischen Eigenschaftsdaten den voreingestellten Bereich nicht überschreiten.

9. Der Funkfrequenzhost gemäß Anspruch 8, wobei der Schritt des Anpassens der Funkfrequenzleistung, wenn die physikalischen Eigenschaftsdaten den voreingestellten Bereich überschreiten, die folgenden Schritte umfasst:
Reduzieren der Funkfrequenzausgangsleistung auf eine voreingestellte erste Zielleistung wenn festgestellt wird, dass die Temperatur oder die Impedanz des in Echtzeit erfassten Objekts größer ist als der Maximalwert des voreingestellten Bereichs; und
Erhöhen der Funkfrequenzausgangsleistung auf eine voreingestellte zweite Zielleistung, wenn festgestellt wird, dass die Temperatur und die Impedanz des in Echtzeit erfassten Objekts beide geringer sind als der Minimalwert des voreingestellten Bereichs.

10. Der Funkfrequenzhost gemäß Anspruch 8, wobei wenn es sich bei einer Funkfrequenzsonde des Funkfrequenzhosts um eine Multielektroden-Funkfrequenzsonde handelt, der Schritt des Anpassens der Funkfrequenzausgangsleistung, wenn die physikalischen Eigenschaftsdaten den voreingestellten Bereich überschreiten, die folgenden Schritte umfasst:
Bestimmen der Gesamtleistung, die gemäß der Minimalimpedanz der Elektrode der Multielektroden-Funkfrequenzsonde anzupassen ist, wenn festgestellt wird, dass die Temperatur oder die Impedanz des in Echtzeit erfassten Objekts höher ist als der Maximalwert des voreingestellten Bereichs;
Erfassen einer Echtzeit-Gesamtleistung der Funkfrequenzsonde des Funkfrequenzhosts, Berechnen einer Leistungsanpassungsmenge durch einen Standard-PID-Algorithmus gemäß der einzustellenden Gesamtleistung und der Echtzeit-Gesamtleistung, und Berechnen der Zielleistung gemäß der Leistungsanpassung und der gegenwärtigen Ausgangsleistung des Funkfrequenzsignals; und
Reduzieren der Funkfrequenz-Ausgangsleistung auf die Zielleistung.

11. Der Funkfrequenzhost gemäß Anspruch 8, wobei der Schritt des Anpassens des voreingestellten Bereichs gemäß der physikalischen Eigenschaftsdaten, die in Echtzeit in einem voreingestellten Zeitraum vor einem gegenwärtigen Moment erkannt werden, umfasst:
Auswählen von Zielwerten aus unterschiedlichen Temperaturwerten und/oder Impedanzwerten um Extremwerte des voreingestellten Bereichs gemäß der unterschiedlichen Temperaturwerte und/oder Impedanzwerte zu aktualisieren, die in Echtzeit in dem voreingestellten Zeitraum erkannt wurden und gemäß einem Standard-Auswahlalgorithmus.

12. Der Funkfrequenzhost gemäß einem der Ansprüche 8 bis 11, wobei der Schritt des Erfassens eingestellter Leistungsdaten entsprechend dem Funkfrequenzbetrieb die folgenden Schritte umfasst:
Erfassen historischer Funkfrequenzbetriebsdaten gemäß der Aufgabe und dem Zweck des Funkfrequenzbetriebs; und
Bestimmen der Ausgangsleistung des Funkfrequenzsignals in den historischen Funkfrequenzbetriebsdaten als die eingestellten Leistungsdaten, wobei es sich bei den eingestellten Leistungsdaten um eine Änderungstrendkurve handelt, die das entsprechende Verhältnis zwischen der Funkfrequenzbetriebszeit und der Ausgangsleistung darstellt.

13. Der Funkfrequenzhost gemäß Anspruch 12, wobei der Schritt des Festlegens einer Ausgangsleistung eines Funkfrequenzsignals gemäß der eingestellten Leistungsdaten umfasst:
Erfassen der Ausgangsleistung in einer Betriebszeit, die dem gegenwärtigen Stand des gegenwärtigen Funkfrequenzbetriebs der Änderungstrendkurve entspricht, und Festlegen der erfassten Ausgangsleistung als die Ausgangsleistung des Funkfrequenzsignals.

14. Der Funkfrequenzhost gemäß einem der Ansprüche 8 bis 11, wobei der Schritt des Erfassens der eingestellten Leistungsdaten gemäß dem Funkfrequenzbetrieb umfasst:
Erfassen von eingestellten Leistungsdaten aus einem externen verbundenen entfernbaren Speicher, oder Erfassen der eingestellten Leistungsdaten die in ein Eingabegerät eingegeben sind, wobei es sich bei den eingestellten Leistungsdaten um ein numerisches Intervall einschließlich eines Maximalwerts der eingestellten Leistung und eines Minimalwerts der eingestellten Leistung handelt.

## Revendications

1. Hôte de radiofréquence, comprenant :
un module d'acquisition (401) configuré pour acquérir des données de puissance de consigne correspondant à une opération de radiofréquence ;
un module de transmission (402) configuré pour régler une puissance de sortie d'un signal de radiofréquence en fonction des données de puissance de consigne, et pour délivrer le signal de radiofréquence à un objet de l'opération de radiofréquence ;
un module de détection (403) configuré pour détecter des données de caractéristiques physiques de l'objet en temps réel, et pour déterminer si les données de caractéristiques physiques dépassent une plage prédéfinie, les données de caractéristiques physiques comprenant la température et/ou l'impédance de l'objet, et la plage prédéfinie étant un intervalle numérique défini par une valeur minimale et une valeur maximale ; et
un module d'ajustement (404) configuré pour ajuster la puissance de sortie de radiofréquence lorsque les données de caractéristiques physiques dépassent la plage prédéfinie, les données de caractéristiques physiques étant considérées comme dépassant la plage prédéfinie lorsqu'elles sont supérieures à la valeur maximale de la plage prédéfinie ou inférieures à la valeur minimale de la plage prédéfinie, et
pour sélectionner des valeurs cibles parmi différentes valeurs de température et/ou valeurs d'impédance afin de réactualiser la valeur maximale et la valeur minimale de la plage prédéfinie en fonction des données de caractéristiques physiques détectées en temps réel pendant une période prédéfinie précédant un moment présent et d'un algorithme de sélection par défaut, lorsque les données de caractéristiques physiques ne dépassent pas la plage prédéfinie.

2. Hôte de radiofréquence selon la revendication 1, dans lequel le module d'ajustement (404) est en outre configuré pour réduire la puissance de sortie de radiofréquence à une première puissance cible prédéfinie lorsque la température ou l'impédance de l'objet détectée en temps réel est supérieure à la valeur maximale de la plage prédéfinie ; et pour augmenter la puissance de sortie de radiofréquence à une deuxième puissance cible prédéfinie lorsque la température et l'impédance de l'objet détectées en temps réel sont toutes deux inférieures à la valeur minimale de la plage prédéfinie.

3. Hôte de radiofréquence selon la revendication 1, dans lequel, lorsqu'une sonde de radiofréquence disposée sur l'hôte de radiofréquence est une sonde de radiofréquence à électrodes multiples, le module de détection (403) est en outre configuré pour déterminer la puissance totale à régler en fonction de l'impédance minimale de l'électrode de la sonde de radiofréquence à électrodes multiples si la température ou l'impédance de l'objet détectée en temps réel est supérieure à la valeur maximale de la plage prédéfinie, et
pour détecter la puissance totale en temps réel de la sonde de radiofréquence de l'hôte de radiofréquence ; et
le module d'ajustement (404) est en outre configuré pour calculer une valeur d'ajustement de puissance au moyen d'un algorithme PID par défaut en fonction de la puissance totale à régler et de la puissance totale en temps réel, pour calculer une puissance cible en fonction de la valeur d'ajustement de puissance et de la puissance de sortie actuelle du signal de radiofréquence, et pour réduire la puissance de sortie de radiofréquence à la puissance cible.

4. Hôte de radiofréquence selon la revendication 1, dans lequel le module d'acquisition (401) est en outre configuré pour acquérir des données historiques d'opération de radiofréquence correspondant à la tâche et à l'objet de l'opération de radiofréquence ; et pour déterminer la puissance de sortie du signal de radiofréquence dans les données historiques d'opération de radiofréquence comme étant les données de puissance de consigne, les données de puissance de consigne consistant en une courbe d'évolution représentant la relation de correspondance entre le temps d'opération de radiofréquence et la puissance de sortie.

5. Hôte de radiofréquence selon la revendication 4, dans lequel le module de transmission (402) est en outre configuré pour acquérir la puissance de sortie à un moment de fonctionnement correspondant à l'étape actuelle de l'opération de radiofréquence en cours, et pour régler la puissance de sortie acquise comme étant la puissance de sortie du signal de radiofréquence.

6. Hôte de radiofréquence selon la revendication 1, dans lequel le module d'acquisition (401) est en outre configuré pour acquérir les données de puissance de consigne à partir d'un support de stockage amovible connecté extérieurement, ou pour acquérir les données de puissance de consigne entrées à partir d'un dispositif d'entrée, les données de puissance de consigne consistant en un intervalle numérique incluant une valeur maximale de la puissance de consigne et une valeur minimale de la puissance de consigne.

7. Hôte de radiofréquence selon la revendication 1, dans lequel le module de transmission (402) est en outre configuré pour régler une valeur médiane de l'intervalle numérique comme puissance de sortie du signal de radiofréquence.

8. Hôte de radiofréquence, comprenant :
un support de stockage (300) et un processeur (400), dans lequel
le support de stockage (300) stocke un code de programme exécutable ; et
le processeur (400) est couplé au support de stockage (300) et configuré pour appeler le code de programme exécutable stocké dans le support de stockage (300), et pour mettre en œuvre un procédé d'ajustement de données comprenant les étapes suivantes :
l'acquisition de données de puissance de consigne correspondant à l'opération de radiofréquence, le réglage d'une puissance de sortie d'un signal de radiofréquence en fonction des données de puissance de consigne, et la délivrance du signal de radiofréquence à un objet de l'opération de radiofréquence ;
la détection de données de caractéristiques physiques de l'objet en temps réel, et la détermination si les données de caractéristiques physiques dépassent une plage prédéfinie, les données de caractéristiques physiques comprenant une température et/ou une impédance de l'objet, et la plage prédéfinie étant un intervalle numérique défini par une valeur minimale et une valeur maximale ;
l'ajustement de la puissance de radiofréquence lorsque les données de caractéristiques physiques dépassent la plage prédéfinie, les données de caractéristiques physiques étant considérées comme dépassant la plage prédéfinie lorsqu'elles sont supérieures à la valeur maximale de la plage prédéfinie ou inférieures à la valeur minimale de la plage prédéfinie, et
la sélection de valeurs cibles parmi différentes valeurs de température et/ou valeurs d'impédance afin de réactualiser des valeurs extrêmes de la plage prédéfinie en fonction des données de caractéristiques physiques détectées en temps réel pendant une période prédéfinie précédant un moment présent et d'un algorithme de sélection par défaut lorsque les données de caractéristiques physiques ne dépassent pas la plage prédéfinie.

9. Hôte de radiofréquence selon la revendication 8, dans lequel l'étape d'ajustement de la puissance de radiofréquence lorsque les données de caractéristiques physiques dépassent la plage prédéfinie comprend les étapes suivantes :
la réduction de la puissance de sortie de radiofréquence à une première puissance cible prédéfinie lorsque la température ou l'impédance de l'objet détectée en temps réel est supérieure à la valeur maximale de la plage prédéfinie ; et
l'augmentation de la puissance de sortie de radiofréquence à une deuxième puissance cible prédéfinie lorsque la température et l'impédance de l'objet détectées en temps réel sont toutes deux inférieures à la valeur minimale de la plage prédéfinie.

10. Hôte de radiofréquence selon la revendication 8, dans lequel lorsqu'une sonde de radiofréquence de l'hôte de radiofréquence est une sonde de radiofréquence à électrodes multiples, l'étape d'ajustement de la puissance de sortie de radiofréquence lorsque les données de caractéristiques physiques dépassent la plage prédéfinie comprend les étapes suivantes :
la détermination de la puissance totale à régler en fonction de l'impédance minimale de l'électrode de la sonde de radiofréquence à électrodes multiples lorsque la température ou l'impédance de l'objet détectée en temps réel est supérieure à la valeur maximale de la plage prédéfinie ;
la détection de la puissance totale en temps réel de la sonde de radiofréquence de l'hôte de radiofréquence,
le calcul d'une valeur d'ajustement de puissance au moyen d'un algorithme PID par défaut en fonction de la puissance totale à régler et de la puissance totale en temps réel, et le calcul de la puissance cible en fonction de l'ajustement de puissance et de la puissance de sortie actuelle du signal de radiofréquence ; et
la réduction de la puissance de sortie de radiofréquence à la puissance cible.

11. Hôte de radiofréquence selon la revendication 8, dans lequel l'étape d'ajustement de la plage prédéfinie en fonction des données de caractéristiques physiques détectées en temps réel pendant une période prédéfinie précédant un moment présent comprend :
la sélection de valeurs cibles parmi différentes valeurs de température et/ou valeurs d'impédance afin de réactualiser des valeurs extrêmes de la plage prédéfinie en fonction des différentes valeurs de température et/ou valeurs d'impédance détectées en temps réel pendant la période prédéfinie et d'un algorithme de sélection par défaut.

12. Hôte de radiofréquence selon l'une quelconque des revendications 8 à 11, dans lequel l'étape d'acquisition de données de puissance de consigne correspondant à l'opération de radiofréquence comprend les étapes suivantes :
l'acquisition de données historiques d'opération de radiofréquence correspondant à la tâche et à l'objet de l'opération de radiofréquence ; et
la détermination de la puissance de sortie du signal de radiofréquence dans les données historiques d'opération de radiofréquence comme étant les données de puissance de consigne, les données de puissance de consigne consistant en une courbe d'évolution représentant une relation de correspondance entre le temps d'opération de radiofréquence et la puissance de sortie.

13. Hôte de radiofréquence selon la revendication 12, dans lequel l'étape de réglage d'une puissance de sortie d'un signal de radiofréquence en fonction des données de puissance de consigne comprend :
l'acquisition, à partir de la courbe d'évolution, de la puissance de sortie à un moment de fonctionnement correspondant à l'étape actuelle de l'opération de radiofréquence en cours, et le réglage de la puissance de sortie acquise comme étant la puissance de sortie du signal de radiofréquence.

14. Hôte de radiofréquence selon l'une quelconque des revendications 8 à 11, dans lequel l'étape d'acquisition de données de puissance de consigne correspondant à l'opération de radiofréquence comprend :
l'acquisition des données de puissance de consigne à partir d'un support de stockage amovible connecté extérieurement, ou l'acquisition des données de puissance de consigne entrées à partir d'un dispositif d'entrée, les données de puissance de consigne consistant en un intervalle numérique incluant une valeur maximale de la puissance de consigne et une valeur minimale de la puissance de consigne.
